# EUROPEAN PATENT APPLICATION

(11) **EP 3 482 726 A1**
(43) Date of publication of application: **15.05.2019**
(21) Application number: 17425113.2
(22) Date of filing: 14.11.2017
(51) Int. Cl.: A61F 5/03

(54) **HYPER-EXTENDING ORTHOPEDIC BUST**

(71) Applicant: Variteks Italia S.A.S., 98051 Barcellona P.G. (ME) (IT)
(72) Inventor: DE PASQUALE, Carmelo, 98051 Barcellona P.G. (ME) (IT)
(74) Representative: Garavelli, Paolo

(57) **Abstract**

A hyper-extending orthopedic bust (1) is described, composed of a rigid structure comprising a pelvic belt (A) connected through side risers (B) and (B') to a sternal-clavicular band (C) and a back belt (D), the pelvic belt (A) with low horizontal development composed of a pair of lower side bands (2) connected at an adjustable distance through a central lower band (20), each side risers (B) and (B') composed of a lower side riser (4) sliding with respect to a upper side riser (5, 17), the sternal-clavicular band (C) with high horizontal development composed of a pair of upper side bands (13) connected at an adjustable distance through a central upper band (15), the back belt (D) applied to the torso composed of a dorsal-lumbar plaque (10) crossed by a rigid belt (9), the hyper-extending orthopedic bust (1) comprising a first and a second oscillating system to regulate a disposition respectively of the pelvic belt (A) and of the sternal-clavicular band (C) with respect to the side risers (B) and (B').

## Description

The present invention refers to a hyper-extending orthopedic bust.

In general, the present invention refers to orthopedic devices.

In particular, the present invention refers to orthopedic corsets having pressing elements connected in a frame.

A hyper-extending orthopedic bust is a tool used in treating vertebral and dorsal-lumbar pathologies causes by traumas/fractures.

For the above pathologies, the hyper-extending bust indirectly intervenes on the rachis through a rigid apparatus, which stabilizes the spinal column in patients subjected to tumor pathologies, metastases, fractures, vertebral collapses due to osteoporosis, etc., which have to use a support to anti-gravitation muscles.

The three-point hyper-extending bust is a solution to the dorsal-lumbar problems of a patient to allow keeping his position erect through a rigid caging of his torso, which locates two front thrusting points, one applied to the sternum and one to the pelvis, which are counter-posed to a third point in the rear part, precisely in the dorsal-lumbar area.

The usual components of the thereby described bust can be: a lightweight rigid skeleton, made of aluminum or similar alloys, with sternum/clavicular and pelvic bearing; a third dorsal-lumbar thrusting point fastened through a belt; a series of paddings with rigid core, which allow expanding the contact area of the three tension points.

The arrangements which affect the application of a hyper-extending bust are linked to the morphologic features of the patient, adapting his size to the width of his rib cage and the height of his sternum, to the height development of the torso, to the need of a seating action and to the maneuverability when fastening and unfastening the belt.

These regulation and adaptation interventions of the bust on patients with different sizes are currently performed by a technician/physician through screw-type connecting and fastening systems capable of ensuring the perfect alignment of the bust parts symmetrically along the sagittal plane. A well aligned disposition of the bust removes the danger of misalignment induced by accidental force actions and damaging actions of the mechanical parts.

Once worn by the patient, the bust exerts a corrective force of the mechanical type, allowing a mutual patient/bust adaptation and modifying almost immediately the volumes of the patient by hyper-extension. It results that, after a short time of use, the bust disposition must be again modified by a specialist in his orthopedic workshop and through the exclusive use of screwdrivers, causing further stress to the patient, who must go away from the place of his convalescence to perform his due sessions, so that the bust efficiency is constant in time.

The prior art is given by patent US2582930A dealing with a bust comprising right and left parts in contact with rigid supporting portions, rigid sternum and pubic supporting parts, a lumbar supporting portion having continuous flexibility in all the region above the group of lumbar muscles. The lumbar supporting portion comprises a horizontal flexible band substantially not sliding, with means which allow the regulation of its effective length. Moreover, next to a rear opening with three hyper-extension points, the bust comprises rigid pads and a flexible lumbar pad, the rigid panels being stiffly interconnected to a frame comprising independent regulating means.

In spite of these efforts, a need is felt for improving the arrangements which affect the application of a hyper-extending bust depending on the morphologic features of a patient.

An unsolved problems deals with the difficulty of adapting the bust size to the patient, following a series of regulating interventions, which need tiring sessions in the orthopedic workshop of the technician/physician, through interventions on tightening systems with screw connections to mechanically block the parts worn by the patient, in a not always easy way, through the difficult use of cumbersome tools such as the screwdrivers.

Object of the present invention is solving the above prior art problems, by providing a hyper-extending orthopedic bust capable of easily interacting with the patient during the regulation maneuvers along the anatomical profile of the patient.

A further object is establishing an interactive relationship between orthopedic bust and patient to be able to transmit to the patient a constant perception of well-being, following a modification of the arrangement of the orthopedic bust through a regulation performed autonomously.

A further object is reducing to a minimum the necessary mechanisms for regulating the disposition of the orthopedic bust, introducing blocking and regulating systems which are simple, lightweight, efficient and easily and immediately usable both by a technician and by a patient.

A further object is making the patient independent from the need of regulating the disposition of the orthopedic bust when there is a technician.

A further object is setting a bust/patient relationship of a biomechanical nature, which does without the traditional mechanical relationship, to be able to make the patient receptive to the continuous needs of his bust, modulating again its disposition depending on the pathology progresses, due to its simple and practical use, the technician role non being deleted, but minimized to the strictly necessary steps for guaranteeing a correct use of the bust.

The above and other objects and advantages of the invention, as will appear from the following description, are obtained with a hyper-extending orthopedic bust as claimed in claim 1. Preferred embodiments and non-trivial variations of the present invention are the subject matter of the dependent claims.

It is intended that all enclosed claims are an integral part of the present description.

It will be immediately obvious that numerous variations and modifications (for example related to shape, sizes, arrangements and parts with equivalent functionality) can be made to what is described, without departing from the scope of the invention as appears from the following claims.

The present invention will be better described by some preferred embodiments thereof, provided as a non-limiting example, with reference to the enclosed drawings, in which:
Figure 1 shows a diagram of the main parts of an embodiment of the hyper-extending orthopedic bust according to the present invention;
Figures 2, 3 show a perspective view of an embodiment of the hyper-extending orthopedic bust according to the present invention;
Figure 4 shows a front view of an embodiment of the hyper-extending orthopedic bust according to the present invention;
Figure 5 shows a portion V of Figure 2; Figure 6 shows the previous figure in an exploded view;
Figure 7 shows a portion VII from a different perspective with respect to that of Figure 2;
Figure 8 shows the previous figure in a further operating configuration;
Figure 9 shows a side view of a portion IX of Figure 4;
Figure 10 shows a perspective view of a component of Figure 7;
Figure 11 shows an exploded perspective view of a first part of the hyper-extending orthopedic bust according to the present invention;
Figure 12 shows an exploded perspective view of a second part of the hyper-extending orthopedic bust according to the present invention;
Figure 13 shows an exploded perspective view of a third part of the hyper-extending orthopedic bust according to the present invention;
Figure 14 shows a perspective view of the previous figure;
Figure 15 shows a perspective view in a first configuration of a further part of the hyper-extending orthopedic bust according to the present invention; and
Figure 16 shows a perspective view in a second configuration of the previous figure.

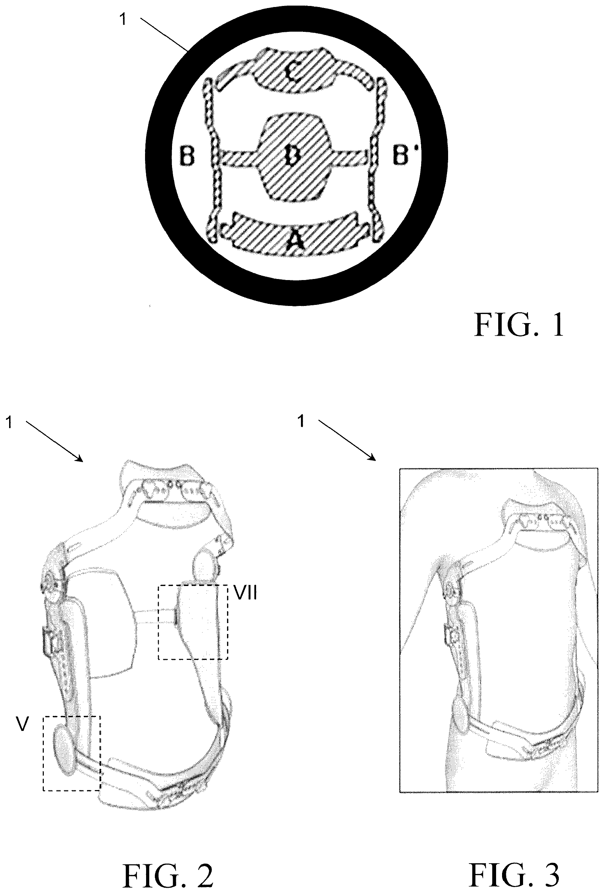

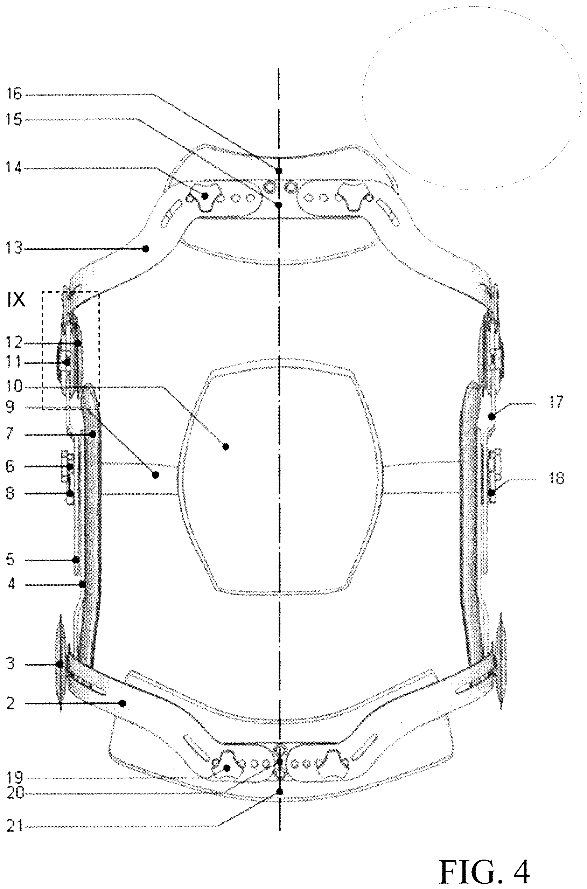

With reference to Figures 1 to 4, it is possible to note that a hyper-extending orthopedic bust 1 is composed of a rigid structure comprising a pelvic belt A connected through side risers B and B' to a sternal-clavicular band C and to a back belt D.

The pelvic belt A with low horizontal development is composed of a pair of lower side bands 2 connected at an adjustable distance through a central lower band 20.

Each side risers B and B' is composed of a lower side riser 4 sliding with respect to an upper side riser 5, 17. The sternal-clavicular band C with high horizontal development is composed of a pair of upper side bands 13 connected at an adjustable distance through a central upper band 15. The back belt D applied to the torso is composed of a dorsal-lumbar plaque 10 crossed by a rigid belt 9.

Advantageously, a first and a second oscillating system allow regulating the disposition respectively of the pelvic belt A and of the sternal-clavicular band C with respect to the side risers B and B'.

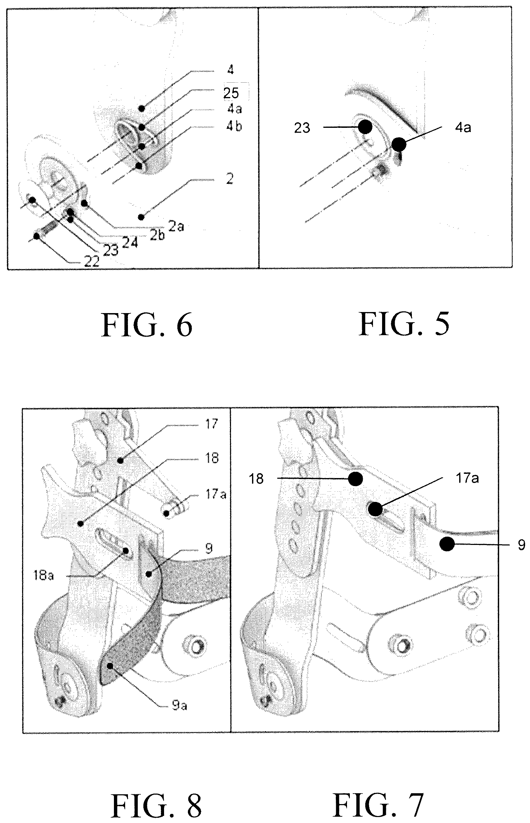

With reference to Figures 5 and 6, the first oscillating system to regulate the disposition of the pelvic belt A with respect to the side risers B and B' comprises a junction between each lower side band 2 and the respective lower side riser 4 having a rotation axis passing along a threaded pin 23, a second pin 4a integral with the lower side riser 4 sliding along a slit 2a obtained in each lower side band 2, in order to limit the rotation angle of the pelvic belt A. A torsion spring 25 is arranged between each lower side riser 4 and the lower side band 2 fastened along the rotation axis through a pin 4b. Blocking of the oscillating movement occurs with the help of a pin 22 screwed to the band 2 through suitable threaded drilling 2b.

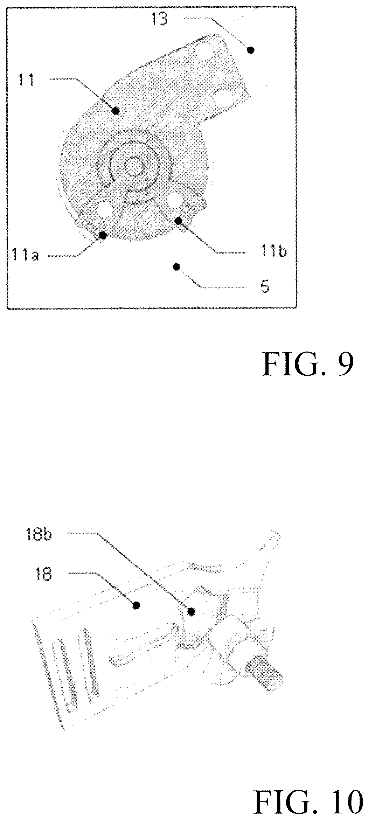

With reference to Figure 9, the second oscillating system to regulate the disposition of the sternal-clavicular band C with respect to the side risers B and B' comprises a junction between each upper side band 13 and the respective upper side riser 5, 17 having an articular clock 11 equipped with flexure regulators 11a and extension regulators 11b to modulate the hyper-extension on the front plane of the sternal-clavicular band C.

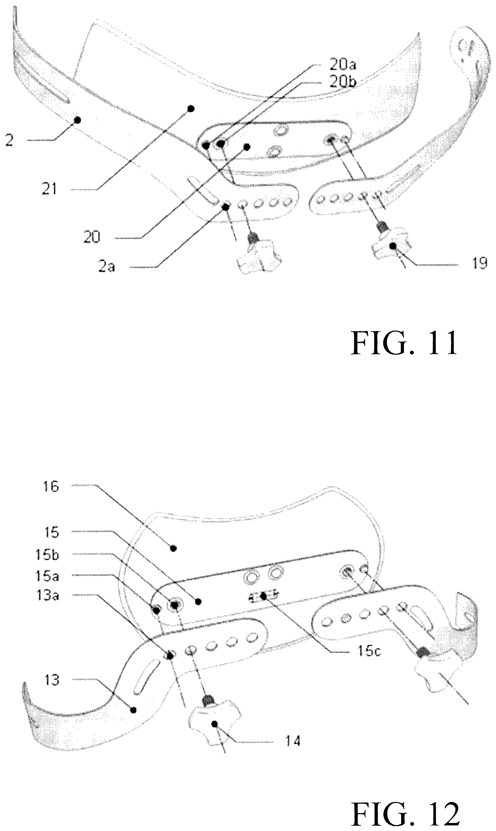

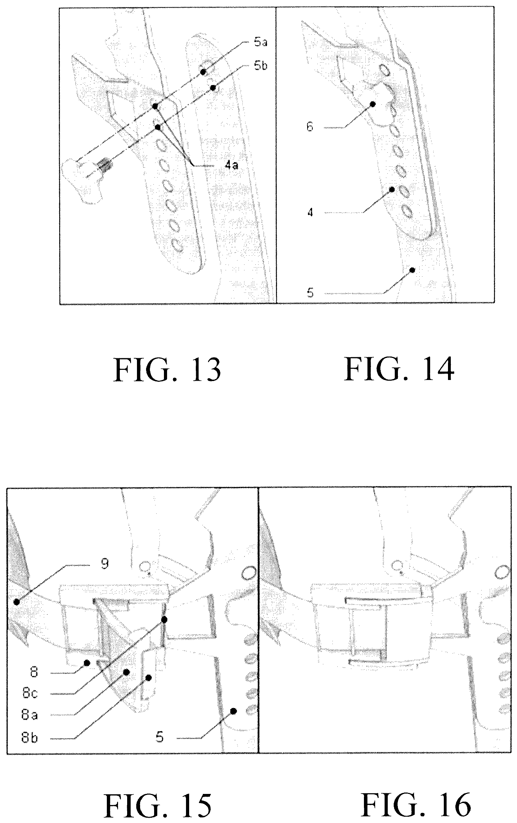

With reference to Figures 11, 12, 13, 14, the connection between the pair of lower side bands 2 and the central lower band 20, the pair of upper side bands 13 and the central upper band 15 and between the lower side risers 4 and the respective upper side riser 5, 17 occurs through a hole blocking system 2a, 13a, 4a which respectively house a pin 20a, 15a, 5a. A screw-type insert 19, 14, 6 screwed in a threaded hole 20b, 15b, 5b allows making monolithic the behavior respectively of the pelvic belt A, of the sternal-clavicular band C and of the risers B, B'.

With reference to Figure 12, a padded plaque 16 is connected to the band 15 through an oscillating system 15c, which abuts onto the sternum to allow inclining and possibly adjusting its position.

With reference to Figures 7 and 8, the belt 9 is connected to the upper riser 17 through an element 18 equipped with a hole 18a with slits adapted to house a pin 17a integral with the upper riser 17. The element 18 is equipped with slits to allow sliding and regulating the rigid belt 9.

With reference to Figure 10, the element 18 has such a shape as to reproduce a lever 18b engaged with each screw-type insert 19, 14, 6 during the regulating step.

The hyper-extending orthopedic bust of the present invention allows performing, through a better practicality, the regulating maneuvers along the anatomic profile of the patient, improving the interactive relationship between bust and patient to allow the patient to have a constant perception of the use of the bust.

In particular, the hyper-extending orthopedic bust allows reducing to a minimum: the use of mechanisms which require specific tools; dexterity, force and technical preparation, replacing them with blocking and regulating systems which are simple, lightweight and easily and immediately usable, both by a technician and by a patient, enabling more autonomy for the patient from the technician, to be able to autonomously intervene in case of misalignments.

The hyper-extending orthopedic bust is composed of a rigid structure made of light aluminum or plastic alloys, adjustable in size along sternum, pelvic area and torso. The use of these lightweight alloys returns a product with high resistance, lightweight and safety, and is adapted thereby to be used also for children. A dorsal-lumbar plaque, fastened through a belt, makes the bust integral with the patient and its paddings enable his comfort above all in the step when it is used.

The hyper-extending orthopedic bust has a rigid structure, creating a seamless solution between the side risers, with vertical development, through a sternal-clavicular band, at the sternum height, and a pelvic belt, at the pelvis height, with horizontal development. The thereby composed elements, including the back belt, are developed like mirrors along an axis of symmetry.

The pelvic belt is a lower rigid horizontal band composed of two lower side bands and of the central lower band adjustable depending on sizes. Regulation and stiffening occur through a blocking system obtained through holes which house a pin and a screw inserted into a threaded hole, removing the chance of moving or rotating. The screw tightening occurs with the help of a wrench, housed on the back of an element, which performs the function of blocking the back belt applied to the torso and composed of a dorsal-lumbar plaque crossed by a rigid belt.

The tightening action between the central lower band and both right and left lower side bands makes the behavior of the pelvic belt monolithic. With reference to Figure 11, the comfort is guarantee by a wide padded band 21, connected to the central band 20 and having an ergonomic shape.

The oscillating system of the pelvic belt A inserted in the fastening area between the low horizontal band 2 and the low vertical riser 4 is used to guarantee comfort when seating and/or flexing frontwards. The rotation axis passes along the suitably sized threaded pin 23, a second pin 4a integral with the riser 4 slides inside a slit 2a obtained in the band 2 to limit the rotation angle of the pelvic belt A. A torsion spring 23, arranged between riser 4 and band 2 and fastened along the rotation axis through the pin 4b, applies a constant thrust towards the rest position of the pelvic belt A. The chance of blocking this oscillating movement occurs with the help of the pin 22 screwed to the band 2 through suitable threaded drilling 2b. The rest position places the screwed pin 22 co-axial with the threaded hole 2b and makes a further screwing possible, which, creating at the other end of the slit 2a a second blocking point, will prevent the oscillating action. The pin 22 is shaped in order to enable a manual screwing through simple arrangements, such as the pressure with the padding 3 and/or the application of a recall spring 24, so that an accidental withdrawal is avoided.

The lower side riser 4 rises with a shaped shape at its base to follow the ergonomics of the patient's pelvis by being joined to the upper side riser 5 to form a single telescopic riser B and B', adjustable in height according to the patient's needs. As for the belt A, size regulation and stiffening occur through a blocking system obtained through the holes 4a which house pin 5a and screw 6 inserted into the threaded hole 5b, to remove any change of movement or rotation. Tightening of the screw 6 occurs with the help of a wrench 18b, housed on the back of the mechanism 18. The tightening action between the elements 4 and 5 make the behavior of the riser B and B' monolithic. The comfort is guaranteed by a wide padded band 7, connected to the riser B through Velcro inserts and by the ergonomic shaping of the described pieces.

The end part of the side risers alto 5 is connected to the upper metallic band 13 belonging to the sternal-clavicular band C through an articular clock 11, which allows, through flexure regulators 11a and extension regulators 11b, modulating the sternal-clavicular band C enabling the hyper-extension.

The sternal-clavicular band C, adjustable in size according to the same principle of the pelvic belt A, is composed of two upper metallic bands 13 shaped depending on the ergonomics of the sternum and connected through the plaque 15. Size regulation and stiffening occur through a blocking system obtained through the holes 13a which house the pin 15a and the screw 14 inserted into the threaded hole 15b to remove the change of movement or rotation. Tightening of the screw 14 occurs with the help of a wrench 18b, housed on the back of the mechanism 18. The tightening action between the elements 13 makes the behavior of the sternal-clavicular band C monolithic. The comfort is guaranteed by a suitable padded plaque 16 connected to the band 15 through a simple oscillating system 15c which abuts onto the sternum and favors inclination and possible adjustment of the position.

The rear lumbar belt D comprises the dorsal-lumbar plaque 10, suitably reinforced and padded, supported through a rigid belt 9 which is tightened to regulate the pressure of the plaque with respect to sternum and pelvis. The connection of the rigid belt 9 to the rigid structure of the bust occurs along both the upper side risers 5 and 17. The upper riser 17 houses the element 18 connected to the belt 9 through slits for regulating the length of the belt 9 and equipped with a slotted hole 18a to guide the pin 17a. A system of male/female Velcro connections 9a on the faces of the belt 9, once having fitted the size, prevents its withdrawal. After having performed the main size regulation, the upper riser 5 houses the element 8 acting on the belt 9 with a further tightening action by lowering the element 8a through a key 8b and engaging the element 8a to the stop 8c, guaranteeing the tightening stability and its easy slackening.

The paddings 3, 7 and 12, together with the dorsal-lumbar plaque 10, the padded band 21 and the padded plaque 16, allow filtering the bust/patient contact, preventing skin abrasions and other types of troubles. A further arrangement provides for the possible drenching of the paddings made of cotton sponge with a aloe essence to prevent skin irritations.

## Claims

1. Hyper-extending orthopedic bust (1) composed of a rigid structure comprising a pelvic belt (A) connected through side risers (B) and (B') to a sternal-clavicular band (C) and a back belt (D), the pelvic belt (A) with low horizontal development composed of a pair of lower side bands (2) connected at an adjustable distance through a central lower band (20), each side risers (B) and (B') composed of a lower side riser (4) sliding with respect to an upper side riser (5, 17), the sternal-clavicular band (C) with high horizontal development composed of a pair of upper side bands (13) connected at an adjustable distance through a central upper band (15), the back belt (D) applied to the torso composed of a dorsal-lumbar plaque (10) crossed by a rigid belt (9), **characterized in that** it comprises a first and a second oscillating system to regulate a disposition respectively of the pelvic belt (A) and of the sternal-clavicular band (C) with respect to the side risers (B) and (B').

2. Hyper-extending orthopedic bust (1) according to the previous claim, **characterized in that** the first oscillating system to regulate the disposition of the pelvic belt (A) with respect to the side risers (B) and (B') comprises a junction between each lower side band (2) and the respective lower side riser (4) having a rotation axis passing along a threaded pin (23), a second pin (4a) integral with said lower side riser (4) sliding along a slit (2a) obtained in each of said lower side bands (2) to limit the rotation angle of the pelvic belt (A), a torsion spring (25) arranged between each lower side riser (4) and the lower side band (2) fastened along the rotation axis through a pin (4b), the blocking of the oscillating movement occurring with the help of a pin (22) screwed to the band (2) through suitable threaded drilling (2b).

3. Hyper-extending orthopedic bust (1) according to claim 1, **characterized in that** the second oscillating system to regulate the disposition of the sternal-clavicular band (C) with respect to the side risers (B) and (B') comprises a junction between each upper side band (13) and the respective upper side riser (5, 17) having an articular clock (11) equipped with flexure regulators (11a) and extension regulators (11b) to modulate the hyper-extension on the front plane of the sternal-clavicular band (C).

4. Hyper-extending orthopedic bust (1) according to any one of the previous claims, **characterized in that** the connection between the pair of lower side bands (2) and the central lower band (20), the pair of upper side bands (13) and the central upper band (15) and between the lower side risers (4) and the respective upper side riser (5, 17) occurs through a blocking system of holes (2a, 13a, 4a) which house respectively a pin (20a, 15a, 5a), a screw-type insert (19, 14, 6) screwed into a threaded hole (20b, 15b, 5b) to make the behavior respectively of the pelvic belt (A), of the sternal-clavicular band (C) and of the riser (B), (B') monolithic.

5. Hyper-extending orthopedic bust (1) according to the previous claim, **characterized in that** it comprises a padded plaque (16) connected to the band (15) through an oscillating system (15c) which abuts onto the sternum to allow inclining and possible adjusting the position.

6. Hyper-extending orthopedic bust (1) according to any one of the previous claims, **characterized in that** the rigid belt (9) is connected to the upper riser (17) through an element (18) equipped with a slotted hole (18a) adapted to house a pin (17a) integral with the upper riser (17), said element (18) equipped with slits to allow sliding and regulating the rigid belt (9).

7. Hyper-extending orthopedic bust (1) according to the previous claim, **characterized in that** said element (18) has a shape capable of reproducing a lever (18b), said lever (18b) engaged with each of said screw-type inserts (19), (14), (6) during the regulating step.
